# EUROPEAN PATENT SPECIFICATION

(11) **EP 0 857 044 B1**
(45) Date of publication and mention of the grant of the patent: **02.01.2002**
(21) Application number: 96928356.3
(22) Date of filing: 04.09.1996
(51) Int. Cl.: A61F 4/00, G08B 3/10, G09B 21/00, G08G 1/005

(54) **A VISUAL AND/OR ACOUSTIC AID FOR PERSONAL USE, AND USE OF THE SAME**
VISUELLE UND/ODER AKUSTISCHE HILFE ZUM PERSÖNLICHEN GEBRAUCH, SOWIE GEBRAUCH DIESER HILFE
AIDE VISUELLE ET/OU ACOUSTIQUE POUR USAGE PERSONNEL ET UTILISATION DE CETTE AIDE

(30) Priority: 05.09.1995 DK 97395
(43) Date of publication of application: 12.08.1998
(73) Proprietor: Dansk Service Center, 4690 Haslev (DK)
(72) Inventor: ANDERSEN, Stig, DK-4690 Haslev (DK)
(74) Representative: Wittrup, Flemming
(86) International application number: DK9600369
(87) International publication number: WO9709009

(56) References cited:
- EP-A- 0 326 129
- EP-A- 0 338 997
- EP-A- 0 583 214
- DE-A- 3 418 053
- FR-A- 2 624 005

## Description

The invention concerns a visual and/or acoustic information transfer equipment for personal use, said equipment including a control unit, one or more antennas coupled to said control unit, a transponder to be carried by a person and capable of being energized by the control unit, a message device controllable by said control unit.

The European Patent Application No. 338 997 and US Patent No. 5 409 380 describe systems of the above-mentioned type. In these known systems, a handicapped person is in possession of a transmitter which can transmit signals via infrared signals to a receiver, which is mounted e.g. on traffic lights at crossings controlled by such lights. However, for the system to work, a blind person himself has to activate his transmitter and thereby activate a receiver which can then emit sound signals enabling the blind person to hear when the crossing is clear. Of course, it is necessary for the blind person to know where the receiver is, if he is to activate it, because the transmitter carried by him is to point to precisely the receiver concerned.

Further, there are systems where sound signals are constantly emitted in crossings controlled by lights, said sound signals being controlled in response to the color signal of a traffic light, where red and yellow signals generate a first sound frequency, while green light generates a second sound frequency which is easy to distinguish from the first one. In general, the strength of the sound signals depends on the ambient noise, so that the sound signals are intensified when the ambient noise increases. These last-mentioned systems have been found to have the drawback that they can be a nuisance to persons having their residence in the vicinity of the traffic lights, in particular at night where the ambient noise is relatively small.

Accordingly, the object of the invention is to improve the previously known equipment such that e.g. a blind person or another handicapped person does not have to activate the activation device himself to make it change to a functional position where it transmits signals to an information source.

Another object is to reduce the emission of sound to a minimum.

The object is achieved by a visual and/or acoustic information transfer equipment as defined in claim 1.

This structure provides a system which "does not need tending". The person carrying the activation device thus does not have to take any steps to activate an information source.

If, as stated in claim 2, a control unit includes a delay circuit which is adapted to delay the signals emitted from the message device, it is ensured that the message to the handicapped person may be synchronized with e.g. green light, so that the blind person does not get any information that the road is clear e.g. just before the light signal changes from green to red. This means that the message may be started at the time when the green signal is turned on.

As stated in claim 3, the transponder is adapted to recognize a series of signals from the control unit (10, 13, 20) some of which signals when send back to the control unit is adapted to make the activation device either active or passive..

The equipment may hereby be adapted to various conditions. For example, a visionally impaired or blind person who is to pass a crossing controlled by lights, will need a sound signal. It will also be an advantage for the visionally impaired or blind person to be able to open a door to a bathroom without "having to search for" the handle. On the other hand, a wheel chair user will hardly need to have activated a signal in connection with a crossing of a road, while a door to a bathroom may expediently be opened by means of the invented device.

In an embodiment of the invention, the activation device may have a permanently programmable unit or a freely programmable unit.

The activation device may hereby be adapted to various uses which may involve activation devices of a certain standard type or more specific user-adapted activatable units, which is achieved particularly by using a freely programmable unit.

According to another embodiments of the invention the equipment may be able to effect the surroundings, e.g. control sound signal emission in a road system controlled by traffic lights, activate an electronic locking mechanism for opening or closing doors and/or control a display for visual information, like stated in the claims 5, 6 and 7.

Even further embodiments according to the invention is
an equipment where the activation of the control unit by the activation device while the message signal is emitted, prolongs the duration of the message signal by a span of time which corresponds to a whole new period,
an equipment where the transponder (1, 12, 18) is energized from the control unit via the antenna,
an equipment where the antennas (6) are concealed, e.g. embedded in a road surface, and
an equipment where the activation device is concealed on the person, or is concealed on an article carried by the person, such as a stick, or on a wheel chair.

The invention will now be explained more fully with reference to an example of the invention shown in the drawing, in which
fig. 1 shows the basic structure of a visual and/or acoustic information transfer equipment according to the invention,
fig. 2 schematically shows the equipment of the invention placed at a crossing,
fig. 3 shows the equipment arranged to open a door,
fig. 4 shows the equipment of the invention in connection with activation of a visual information system, while
fig. 5 shows the equipment of the invention in connection with the activation of an acoustic informatics system.

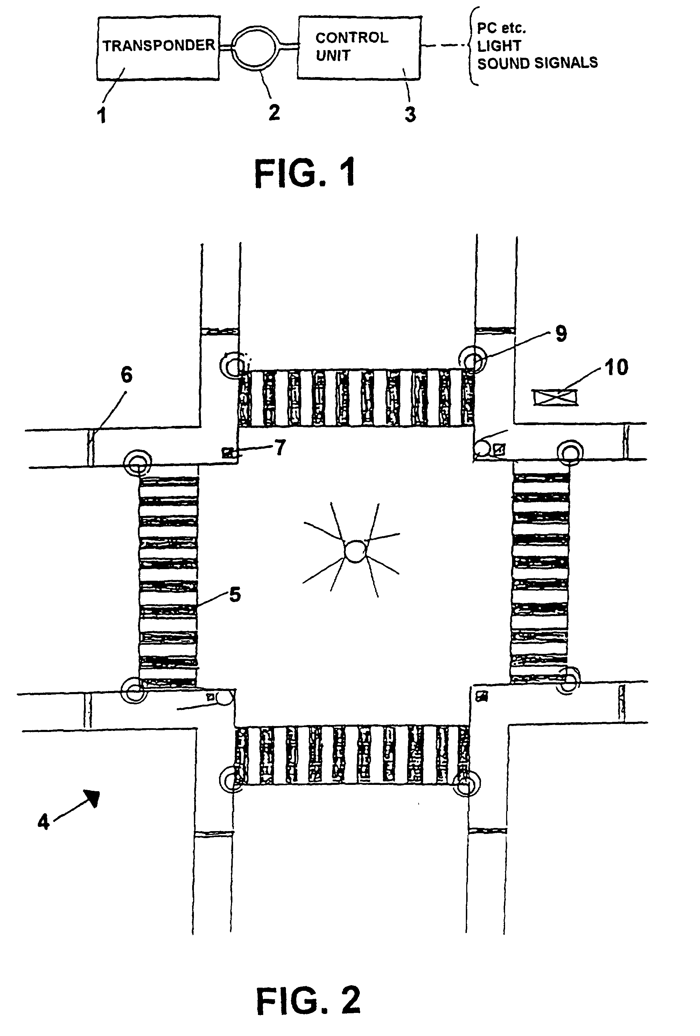

As shown in fig. 1, the equipment of the invention consists of a transponder which is designated by the reference numeral 1, an antenna 2 and a control unit 3.

The basic function of the equipment shown in fig. 1 will now be explained briefly. The control unit 3 currently emits signals via the antenna 2, which can be captured by the transponder 1 when it gets into a region in which the signal strength of the control unit 3 is high enough. The transponder 1 is energized by the control unit 3, and after the transponder 1 has gone into the active state, the control unit 3 will be able to control various devices capable of providing messages of e.g. acoustic/visual type, which may be a tone signal and a synthetic speech signal or even activation signals e.g. to open a door, turn off light, etc.

Fig. 2 shows an equipment used in a crossing controlled by lights. The equipment consists of a plurality of antennas here designated by 6. Expediently, the antennas are concealed in the road, e.g. by being embedded. Further, the drawing shows a plurality of acoustic signal generators having the reference numeral 9. A control unit 10 controls the signals of the antenna and the visual and/or acoustic signal sources, as will be explained more fully below. In some cases, some amplifiers/relays 7 may additionally be provided to avoid radio noise interference, because the signals emitted by the antennas are relatively weak.

The mode of operation of the system is as follows:

If e.g. a blind person wishes to cross the sidewalk 5 in fig. 2, and the person has an equipment according to the invention, a transponder 1, owing to its presence in the crossing 4, will be affected by the control unit 10, which currently emits signals through the antennas 6, and then the transponder 1 assumes an active state so that it can transmit signals through the antennas 6 back to the control unit 10, which can then activate the sound generators 9 in a manner, which will not be described in detail. The equipment is adapted such all the signal generators are affected when a person crosses the pedestrian crossing 5. Further, the system of the invention is operationally reliable in that the signalling is adapted such that the emission of sounds takes place only when the control system of the crossing beings a new periods of green light, so that a passing person will not be "tempted" to enter a crossing where the green light is about to change to red light. Further, a blind person arriving at the crossing in the middle of a sound emission period will make the system begin on a fresh period so that there will be ample time to pass the crossing.

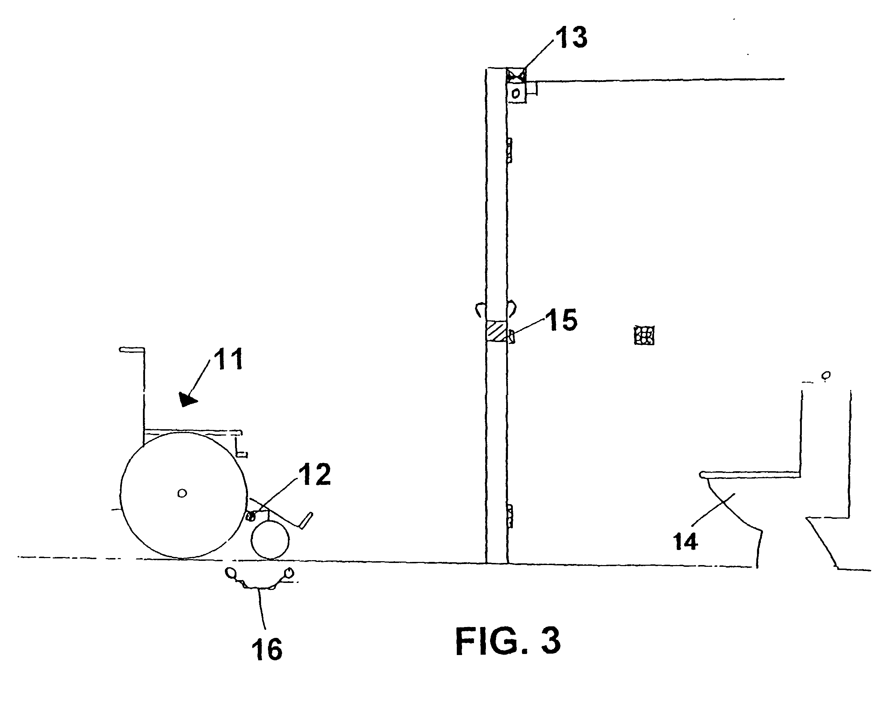

Fig. 3 shows another use of the equipment of the invention. In the figure, 11 designates a wheel chair which is equipped with a transponder 12, which may very well be concealed on the wheel chair 11. Further, the figure shows the antenna 16 which communicates with the control unit 13. The system operates in the manner that when a wheel chair user approaches a door having a lock 15, the transponder 12 will enter into an active state, in which it is affected by the antenna 16, which hereby activates the transponder 12 and transmits a signal back to the control unit 13 ensuring that the lock 15 of the door will be opened by mans of a suitable electronic unlocking mechanism. Further, the door automatically opens. It closes again after a predetermined period of time, so that the handicapped person himself does not have to affect the door mechanically in any manner.

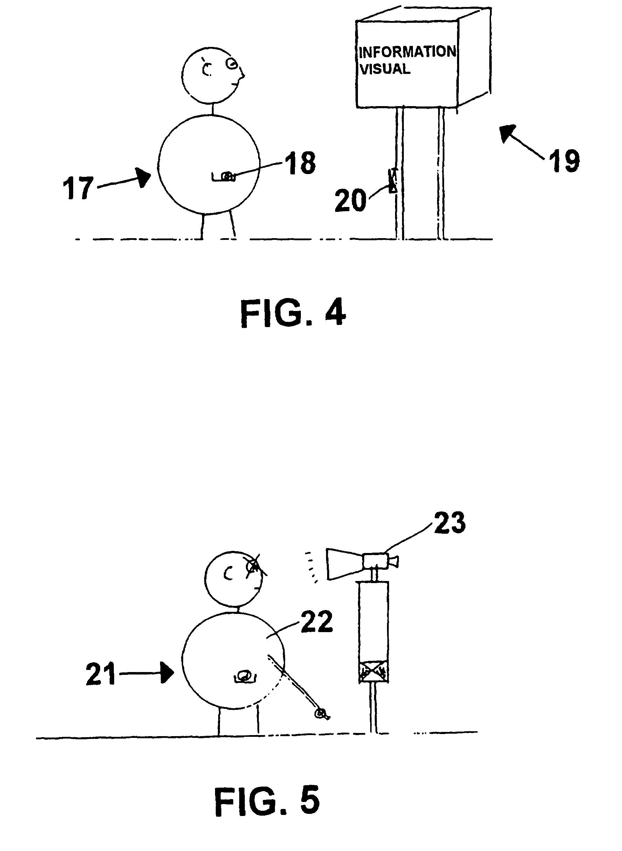

Fig. 4 shows another use of the equipment of the invention, where a person 17 wishes to see a map of the town in which he is present. For this purpose, the person 17 has a transponder 18 which, when it approaches the standard 19 equipped with the control unit 20, turns on the standard for display of visual information.

Fig. 5 shows a system similar to that in fig. 3, but with the difference that it is a sound signal generator 23 which lends itself for use particularly by blind or visually impaired persons. The system operates in the same manner as the system in fig. 4.

Examples of other applications of the equipment include:
- residential homes for persons suffering from dementia senilis, where the equipment alarms the nursing staff if the patient tries to leave a ward/an area of the residential home
- in the hospital sector to identify both beds and patients and thus door opening possibilities/lift reservations with automation
- access control systems.

## Claims

1. A visual and/or acoustic information transfer equipment for personal use, said equipment including a control unit, one or more antennas coupled to said control unit, a transponder to be carried by a person and adapted to be energized by the control unit, a message device controllable by said control unit, **characterized in that** said transponder is adapted to transmit a signal back to the control unit when energized by the control unit, said control unit is adapted to activate the message device (9, 10, 23) in response to said transmitted signal, said message device is adapted to emit an acoustic signal or a visual signal or an activation signal.

2. An equipment according to claim 1, **characterized in that** the control unit is adapted to delay the signals emitted from the message device.

3. An equipment according to claim 1 or 2, **characterized in that** the transponder is adapted to recognize a series of signals from the control unit (10, 13, 20) some of which signals when sent back to the control unit is adapted to make the transponder either active or passive.

4. An equipment according to claim 1 - 3, **characterized in that** the transponder has a permanently programmable unit or freely programmable unit.

5. An equipment according to claim 1 - 4, **characterized in that** the equipment is adapted to control sound signal emission in a road system controlled by traffic lights.

6. An equipment according to claim 1 - 4, **characterized in that** the equipment is adapted to activate an electronic locking mechanism for open or closing a door.

7. An equipment according to claim 1 - 4, **characterized in that** equipment is adapted to control a display for visual information.

## Patentansprüche

1. Ein System zur Übertragung visueller und/oder akustischer Information für den persönlichen Gebrauch, das eine Kontrolleinheit, eine oder mehrere mit dieser Kontrolleinheit verbundene Antennen, einen von einer Person zu tragenden, von der Kontrolleinheit aktivierbaren Antwortsender und eine von dieser Kontrolleinheit steuerbare Mitteilungsvorrichtung einschließt. Das System ist **dadurch gekennzeichnet, dass** der Antwortsender ein Signal zur Kontrolleinheit zurücksenden kann, wenn er durch die Kontrolleinheit aktiviert wird. Die Kontrolleinheit kann auf dieses übertragene Signal dadurch reagieren, dass sie die Mitteilungsvorrichtung (9, 10, 23) aktiviert, die ein akustisches Signal oder ein visuelles Signal oder ein Aktivierungssignal aussenden kann.

2. Ein System gemäß dem Patentanspruch 1, das **dadurch gekennzeichnet ist, dass** die Kontrolleinheit das von der Mitteilungsvorrichtung ausgesandte Signal verzögern kann.

3. Ein System gemäß dem Patentanspruch 1 oder 2, das **dadurch gekennzeichnet ist, dass** der Antwortsender eine Reihe Signale von der Kontrolleinheit (10, 13, 20) wiedererkennen kann, von denen einige den Antwortsender entweder aktiv oder passiv machen können, wenn sie zur Kontrolleinheit zurückgesandt werden.

4. Ein System gemäß den Patentansprüchen 1 - 3, das **dadurch gekennzeichnet ist, dass** der Antwortsender mit einer permanent programmierbaren Einheit oder einer frei programmierbaren Einheit ausgerüstet ist.

5. Ein System gemäß den Patentansprüchen 1 - 4, das **dadurch gekennzeichnet ist, dass** es die Aussendung von Schallsignalen in einem von Verkehrsampeln geregelten Straßensystem steuern kann.

6. Ein System gemäß den Patentansprüchen 1 - 4, das **dadurch gekennzeichnet ist, dass** es eine elektronische Sperrvorrichtung zur Öffnung oder Schließung einer Tür aktivieren kann.

7. Ein System gemäß den Patentansprüchen 1 - 4, das **dadurch gekennzeichnet ist, dass** es eine Anzeige für visuelle Information steuern kann.

## Revendications

1. Un système de transfert d'informations visuelles et/ou acoustiques à usage personnel, ledit système comprenant une unité de contrôle, une ou plusieurs antennes reliées à ladite unité de contrôle, un transpondeur qui doit être porté par une personne et qui peut être actionné depuis ladite unité de contrôle, un dispositif de communication qui peut être commandé par ladite unité de contrôle. Le système est **caractérisé par ce que** ledit transpondeur peut renvoyer un signal à ladite unité de contrôle lorsqu'il est actionné par ladite unité de contrôle. Ladite unité de contrôle peut réagir audit signal envoyé, en actionnant le dispositif de communication (9, 10, 23), qui peut émettre un signal acoustique ou un signal visuel ou un signal d'actionnement.

2. Un système conforme à la revendication 1, **caractérisé par ce que** l'unité de contrôle peut retarder le signal envoyé par le dispositif de communication.

3. Un système conforme aux revendications 1 ou 2, **caractérisé par ce que** le transpondeur peut reconnaître une série de signaux venant de l'unité de contrôle (10, 13, 20), signaux dont quelques-uns peuvent rendre le transpondeur soit actif soit passif lorsqu'ils sont renvoyés à l'unité de contrôle.

4. Un système conforme aux revendications 1 à 3, **caractérisé par ce que** le transpondeur contient une unité programmable de façon permanente ou une unité librement programmable.

5. Un système conforme aux revendications 1 à 4, **caractérisé par ce qu'**il peut contrôler l'envoi de signaux acoustiques dans un système de routes réglé par des feux de signalisation routière.

6. Un système conforme aux revendications 1 à 4, **caractérisé par ce qu'**il peut actionner un mécanisme électronique de verrouillage destiné à l'ouverture ou la fermeture d'une porte.

7. Un système conforme aux revendications 1 à 4, **caractérisé par ce qu'**il peut commander un écran destiné à l'information visuelle.
